# EUROPEAN PATENT SPECIFICATION

(11) **EP 1 875 321 B1**
(45) Date of publication and mention of the grant of the patent: **13.01.2010**
(21) Application number: 06726924.1
(22) Date of filing: 26.04.2006
(51) Int. Cl.: G05D 7/06, G05D 21/02

(54) **FLOW CONTROL**
FLUSSSTEUERUNG
REGULATION D'ECOULEMENT

(30) Priority: 29.04.2005 GB 0508657
(43) Date of publication of application: 09.01.2008
(73) Proprietor: Tristel PLC, Fordham Road, Snailwell Newmarket CB8 7NY (GB)
(72) Inventor: DURRANT, John, Hadleigh, Ipswich IP7 5AP (GB); HAWKER, Michael, John, Hadleigh, Ipswich IP7 5AP (GB)
(74) Representative: Gemmell, Peter Alan
(86) International application number: PCT/GB2006/001542
(87) International publication number: WO 2006/117518

(56) References cited:
- DE-A1- 3 400 263
- GB-A- 1 168 015
- GB-A- 2 175 399
- US-A- 5 970 999
- US-A- 6 042 802
- US-A1- 2004 101 438

## Description

### FIELD OF THE INVENTION

The present invention relates to a method and apparatus for controlling the flow of fluids through a sample channel. The invention has particularly application in medical facilities for measuring or verifying the concentration of an active component of a sterilizing fluid.

### BACKGROUND TO THE INVENTION

Chlorine dioxide (ClO₂) is an important sterilizing agent in medical facilities such as surgeries and hospitals. It is used for sterilizing medical and dental equipment or for wound irrigation or skin asepsis.

Apparatus for generating a stream of aqueous chlorine dioxide sterilizing fluid is described in the patent document WO2005/011759 A1. The apparatus disclosed in this document is able to produce sterilizing fluid which is dosed with ClO₂ in varying quantities depending on the particular sterilizing requirements.

It is important to be able to measure or verify the concentration of the active component in a sterilizing fluid to ensure that the correct sterilizing requirements are being met during the sterilizing process. The apparatus disclosed in W02005/011759 uses a probe capable of directly measuring concentrations of ClO₂ (the active component of the sterilizing fluid) in the range 1-20 ppm.

As discussed in WO2005/011759, probes for directly measuring higher ClO₂ concentrations, for example in the range 100-200 ppm, tend to be far more sensitive and are damaged by prolonged exposure to such high concentrations of ClO₂. Therefore, indirect techniques are used to measure concentrations of ClO₂ in excess of 20 ppm. These indirect techniques involve measuring a diluted sample of the ClO₂-dosed sterilizing fluid 11, and using an algorithm to calculate the concentration of the original sample.

It is far more desirable, however, to be able directly to measure high concentrations and not have to rely on indirect techniques which give rise to greater uncertainties in the results. When using sensitive probes capable of directly measuring high concentrations, it is necessary to limit the time in which the probe is in contact with the sterilizing fluid. It is also advantageous to be able to flush the probe system in-between measurements to remove all traces of the sterilizing fluid. Furthermore, for calibration reasons and to prolong the lifetime of the probe, it is desirable to leave the probe submersed in a neutral fluid in-between measurements.

DE3400263 discloses a device for monitoring the concentration at which disinfectant solutions are prepared from water and concentrated disinfectant by means of a metering device. The device has a conductivity-measuring probe immersed in the disinfectant solution. Electrical signals from the probe are supplied to an evaluation device with which the conductivity is monitored as a measure of the concentration being used.

### SUMMARY OF THE INVENTION

The invention is defined by independent claims 1 and 20. Preferred features are specified in the dependent claims.

Preferably a probe is located within the sample channel. The term 'probe' is intended to be construed broadly, and to mean anything that is capable of detecting or measuring a property of a fluid. The term 'sample channel' is also intended to be construed broadly, and to include any fluid carrying channel in which flow control as described above is achieved. It is not essential that measurements are taken in the sample channel.

Preferably the probe is used to measure the concentration of a component of the first fluid, and the apparatus preferably comprises processing means for converting the probe reading to a concentration value.

Concentration measuring probes tend to require contact with the analyte for an extended period of time before an accurate and stable concentration measurement is obtained. This time period is often of the order of minutes. Ensuring fluid from the reservoir flows past the probe for a number of minutes could require having a large reservoir capable of containing a large volume of fluid. Preferably, however, a return channel is connected between the sample channel and the reservoir to form a recirculation loop. Fluid from the reservoir may circulate around the loop and past the probe a number of times for a predetermined time period long enough for the probe to take an accurate and stable reading. Preferably, this predetermined time period is around two minutes.

In a preferred embodiment, the second fluid is used to flush out the system after the probe has been in contact with the first fluid. The second fluid is preferably a neutral fluid, and most preferably is water. The apparatus is particularly suitable for use in hospitals or dental practices, and may be connected to the hospital's or dental practice's regular supply of tap water. Preferably, deionised water is used to set the zero level of the probe.

The second fluid is preferably pumped through the sample channel and through the return channel into the reservoir to clean the reservoir before filling with the first fluid. The system may, however, comprise a plurality of separate inlets for the second fluid so that the various parts of the system may be flushed with second fluid from various sources.

Preferably the pump means comprises a single pump for pumping fluid out of the reservoir, through the sample channel, back to the reservoir, and out to waste. A plurality of pumps may however be used instead to separately perform the various pumping tasks.

The apparatus may include means for selectively allowing fluid to enter the sample channel or the reservoir. Preferably these means include solenoid valves, however other types of switchable valves could also be used. The means for trapping fluid in the sample channel also preferably comprises two solenoid valves, with the fluid being trapped therebetween.

Preferably the reservoir has a removable lid. A duckbilled valve is preferably located near the top of the reservoir for example in the reservoir lid for admitting air into the reservoir when fluid is pumped from the reservoir. This eases the pumping process, and prevents the pump having to pump against a vacuum. Additionally, the duckbilled valve prevents liquid spilling out of the top of the reservoir if the reservoir is overfilled.

The apparatus is designed to be compatible with many different types of devices. These devices are typically devices for cleaning medical equipment, each of which operates at a different flow rate and pressure. The reservoir will therefore be subject to different fill rates depending upon the device it is incorporated within. The reservoir preferably includes means for controlling the level of fluid in the reservoir to prevent the reservoir from overflowing, for example an overflow outlet. Alternatively, sensing means can be included to sense when a predefined maximum fill level is achieved. The apparatus may be configured such that the solenoid valves prevent additional fluid entering the reservoir when the maximum fill level is reached.

The syphon preferably comprises concentric inner and outer cylinders. It is preferable that the height of the inner cylinder is substantially equal to or greater than the maximum fill level of the reservoir. This arrangement is a safety feature, and ensures that in the event that the airlock in the syphon fails unexpectedly, fluid in the reservoir cannot spill over the inner cylinder walls and enter the sample channel.

Preferably the method includes the step of checking the measured concentration to determine whether or not it is within a predetermined tolerance of the desired concentration. Display means may be used to indicate a 'pass' or 'fail' depending on whether the concentration is within the tolerance or not. The display means could be a display screen or a ticket could be printed.

Other aspects and benefits of the invention will appear in the follow specification, drawings and claims.

### BRIEF DESCRIPTION OF THE DRAWINGS

The invention will now be further described, by way of example, with reference the following drawings in which:
Figure 1 is a schematic drawing showing apparatus for controlling fluid flow in accordance with an aspect of the present invention;
Figures 2 to 9 show the fluid flow in the apparatus of Figure 1 during various stages of operation;
Figure 10 is a view of the region around the reservoir outlet of the apparatus of Figure 1; and
Figure 11 is a flow sheet for a method of producing a stream of aqueous ClO₂ sterilizing fluid in accordance with an aspect of the present invention.

### DETAILED DESCRIPTION

The invention is now described for convenience in relation to an apparatus and method for measuring the concentration of ClO₂ in a sterilizing fluid, the apparatus comprising means for controlling the flow of two fluids. It should be understood this is one particular example of an application of the invention, and that the invention may have application in any field where it is necessary to control the flow of at least two fluids through a single channel whilst keeping the fluids separate and therefore preventing the fluids from mixing.

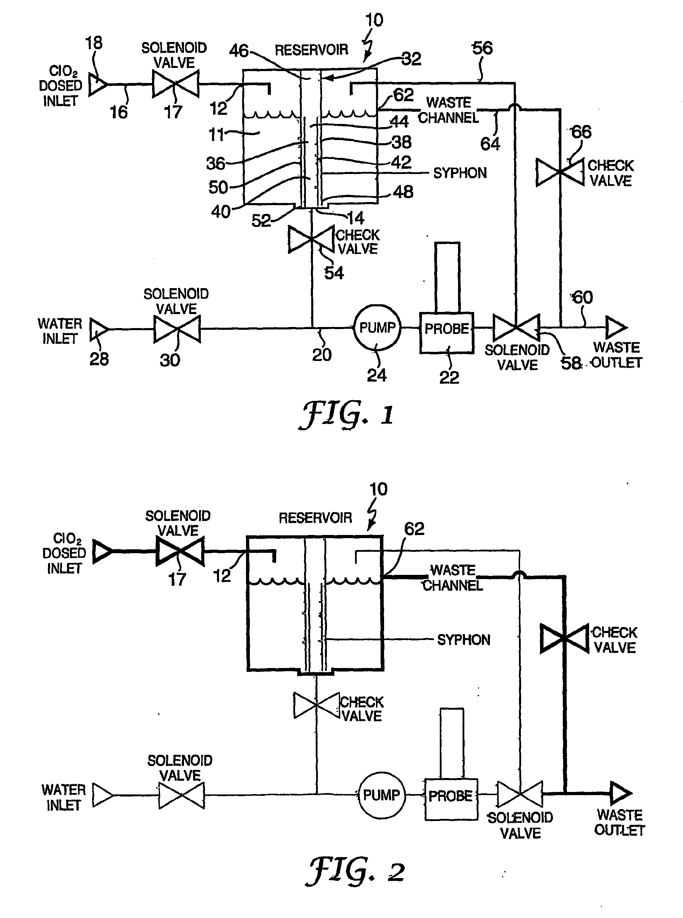

Referring to Figure 1, apparatus is shown for measuring the concentration of a ClO₂-dosed sample of sterilizing fluid. The apparatus includes a reservoir 10 containing sterilizing fluid 11, and having a reservoir inlet 12 and a reservoir outlet 14. The reservoir inlet 12 is connected to a ClO₂-dosed sample supply line 16 having a ClO₂ inlet 18 for connection to a source of ClO₂-dosed sterilizing fluid 11.

A first solenoid valve 17 for selectively allowing ClO₂-dosed sterilizing fluid 11 into the reservoir 10 is disposed in sample supply line 16 upstream of the reservoir inlet 12

The reservoir outlet 14 is connected to a sample channel 20. A probe 22 for measuring the concentration of ClO₂ in the sterilizing fluid 11 is located in the sample channel 20. A pump 24 is also located in the sample channel 20, and is used for pumping fluid through the sample channel 20. In this example, the pump 24 is a KNF pump which has a 6 Bar (0.6 MPa) pressure rating.

The sample channel 20 has a sample -channel inlet 28 for connection to a source of water. A second solenoid valve 30 is provided in the sample channel 20, downstream of the sample channel inlet 28 for selectively allowing water to flow through the sample channel 20. The second solenoid valve could equally usefully be located upstream of the sample channel inlet 28 instead.

A syphon 32 is connected between the reservoir 10 and the sample channel 20. The syphon 32 is located within the reservoir 10 between the fluid 11 and the reservoir outlet 14, such that the fluid in the reservoir must flow through the syphon 32 to reach the reservoir outlet 14.

The syphon 32 comprises an inner cylinder 36 and an outer cylinder 38. The lower end 40 of the inner cylinder 36 surrounds the reservoir outlet 14, and has side walls 42 which extend upwards towards the top of the reservoir 10. The inner cylinder 36 has an open top end 44.

The outer cylinder 38 surrounds the inner cylinder 36. The outer cylinder is upturned and has a sealed top end 46 which is located near the top of the reservoir. The outer cylinder 38 has an open bottom end 48 which is located near the reservoir outlet 14. The outer cylinder 38 has side walls 50 extending from the top of the reservoir 10 towards the bottom of the reservoir 10.

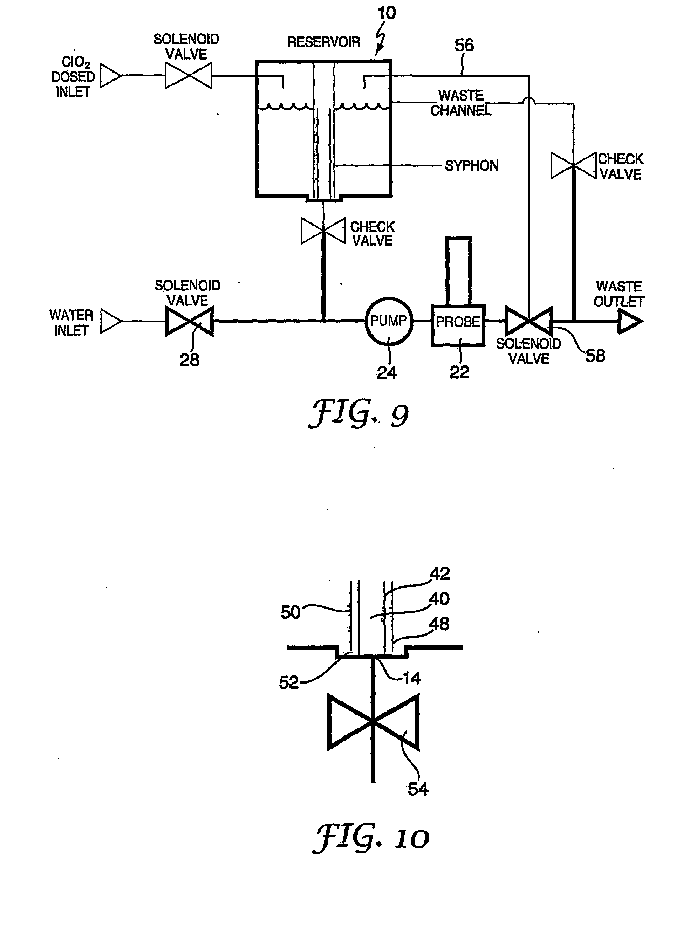

There is a gap 52 between the lower end 48 of the outer cylinder 38 and the bottom of the reservoir 10. The gap 52 allows fluid in the reservoir 10 to enter the syphon 32. The gap is shown more clearly in Figure 10.

A reservoir check valve 54 is located outside the reservoir 10 adjacent the reservoir outlet 14. The reservoir check valve 54 is a one way valve and prevents fluid from entering the reservoir through the reservoir outlet 14.

The embodiment of the invention illustrated in Figure 1 also includes a return channel 56. One end of the return channel 56 is connected to the sample channel 20 downstream of the pump 24. The other end of the return channel 56 feeds into the reservoir 10. The return channel 56 is used for carrying fluid from the sample channel 20 to the reservoir 10.

A third solenoid valve 58 is located at the junction between the sample channel 20 and the return channel 56. The third solenoid valve 58 is used for directing fluid in the -sample channel 20 either through the return channel 56 and into the reservoir 10, or through a waste outlet pipe 60.

An overflow outlet 62 is located in the reservoir 10 to prevent the tank from overflowing. A waste channel 64 connects the overflow outlet 62 to the waste outlet pipe 60. A waste check valve 66 is located in the waste channel 64 for preventing fluid passing from the waste channel 64 into the reservoir 10.

The following describes the principles of operation of the above described apparatus:

Dosed sterilizing fluid 11 from a dosed sterilizing fluid generator (not shown) enters the reservoir 10 through the reservoir inlet 12. Flow through the reservoir inlet 12 is controlled by the first solenoid valve 17. In this example, the reservoir 10 holds about 100 ml of dosed sterili-zing fluid 11. Surplus dosed sterilizing fluid 11 escapes out of the reservoir 10 through the overflow outlet 62. The waste check valve 66 in the waste outlet pipe 64 ensures that liquid can only pass out of, and not into, the reservoir through the waste outlet.

Undosed water enters the system through the water inlet 28. The water inlet 28 is controlled by the second solenoid valve 30. This water is used to flush the reservoir 10, pump 24 and probe 22 to remove traces of ClO₂.

The pump 24 and probe 22 are connected to the reservoir outlet 14. The reservoir check valve 54 ensures that liquid can only be pumped out of, and not into, the reservoir 10. The reservoir outlet 14 is separated from the liquid in the reservoir by the syphon 32. Unless liquid is being pumped out of the reservoir 10, the syphon 32 is filled with air and forms an air lock that separates the contents of the reservoir 10 from the sample channel 20.

The third solenoid valve 58 controls the flow of fluid from the pump 24. Fluid can either be pumped through the return channel 56 and up to the reservoir 10, or out to the waste outlet pipe 60.

The sequence of operation for the apparatus will now be described with reference to Figure 1, and with additional reference to Figures 2-9. In Figures 2-9, heavy black lines are used to highlight the relevant parts of the apparatus used for each stage of operation. Reference numbers in the text refer to the parts as indicated in Figure 1.

### Stage 1: Fill reservoir with dosed sample

Referring to Figure 2, the first solenoid valve 17 is opened and dosed sterilizing fluid 11 is admitted into the reservoir 10. The fill rate depends upon the system flow rate and pressure, and this is generally controlled by the device receiving the dosed sterilizing fluid 11. The system is suitable for use in a range of devices for cleaning endoscopes or other medical equipment. These devices tend to operate at different pressures and flow rates depending on the device type and manufacturer. There is a fixed jet size on the reservoir inlet 12, therefore the volume of dosed sterilizing fluid 11 admitted into the reservoir 10 will typically be in the range of about 40 - 100 ml when the system is used with washers/disinfectant machines such as the Dawmed Clinic (RTM) and the Medivator (RTM). Typical line pressures for these devices are in the range 1.03-4.14 bar (15-60 psi), and typical flow rates are in the range 3-20 litres/minute.

The overflow outlet 62 enables the system to adapt to such different supply pressures and flow rates. Excess of dosed sterilizing fluid 11 flows through the overflow outlet 62 to waste. Preferably, however, the reservoir 10 is filled with at least about 40 ml of dosed sterilizing fluid 11, and wastage is minimised by ensuring that no more than about 100 ml enters the reservoir 10. The first solenoid valve 17 is opened for a sufficient period of time to fill the reservoir 10 with at least 40 ml of dosed sterilizing fluid 11. The flow rate and pressure varies with application, so a minimum time can be set in which the first solenoid valve 17 remains open to ensure at least 40 ml enters the reservoir 10, or the time in which the first solenoid valve 17 remains open can be changed to suit the application.

### Stage 2: Measure ClO₂ concentration

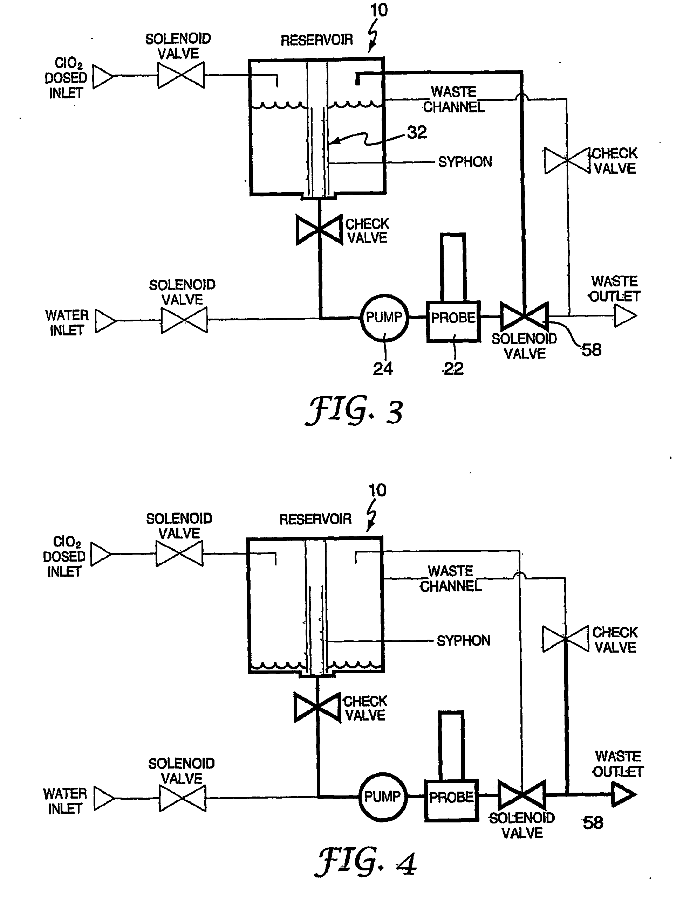

Referring to Figure 3, the third solenoid valve 58 is set to allow fluid to be pumped up to the reservoir 10. The pump 24 is activated to perform the pumping. Air in the syphon 32 is drawn through the pump 24 and probe 22. When the air in the syphon 32 has been evacuated, dosed sterilizing fluid 11 is drawn through the pump 24 and probe 22 and is pumped back up to the reservoir 10 through the return channel 56. Dosed sterilizing fluid 11 is circulated for two minutes to allow the probe 22 to measure the ClO₂ concentration and provide a stable result.

The flow rate is determined by the recommended flow rate across the probe 22 which is defined by the probe manufacturer, but is typically in the range 100 - 200 ml/minute. To achieve such a flow rate, a pump such as a KNF (RTM) diaphragm pump, may be used which operates at a flow rate of 100-150 ml/minute at 24 volts.

### Stage 3: Empty the reservoir

Referring to Figure 4, the third solenoid valve 58 is set to allow liquid to be pumped to waste. The contents of the reservoir 10 are pumped to waste.

### Stage 4: Flush the pump and probe with plain water

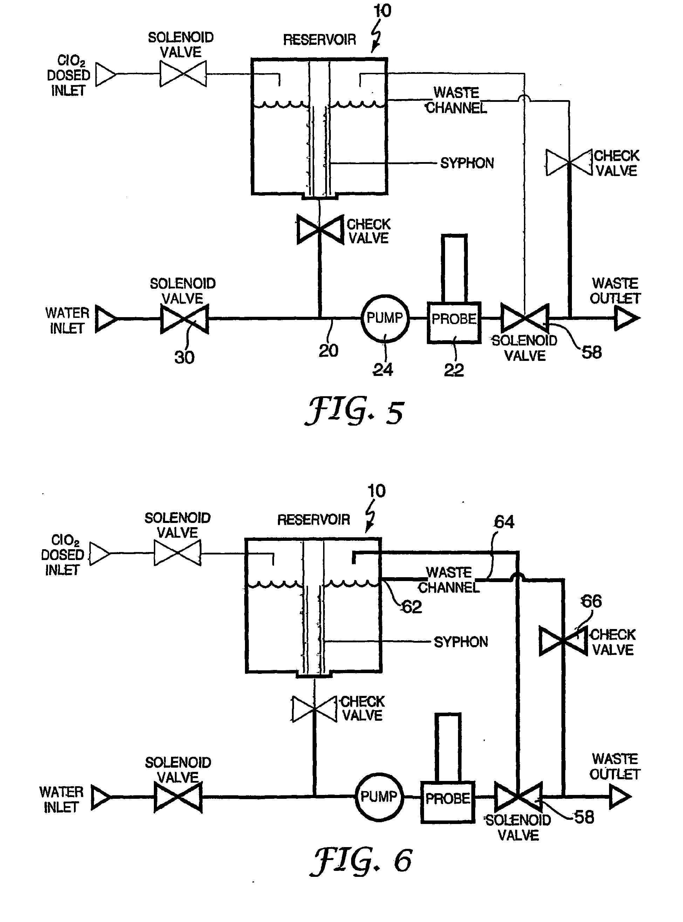

Referring to Figure 5, the second solenoid valve 30 is opened to allow water into the sample channel 20. The third solenoid valve 58 is set to direct fluid to waste and prevent fluid from passing up to the reservoir 10. The pump 24 is used to pump water through the sample channel 20 and through the probe 22 to waste.

### Stage 5: Flush the reservoir and syphon tubes

Referring to Figure 6, the third solenoid valve 58 is set to allow water to be pumped up to the reservoir 10. The reservoir 10 is filled with water and allowed to overflow through the overflow outlet 62, through the waste channel 64 and waste check valve 66 to waste.

### Stage 6: Empty reservoir

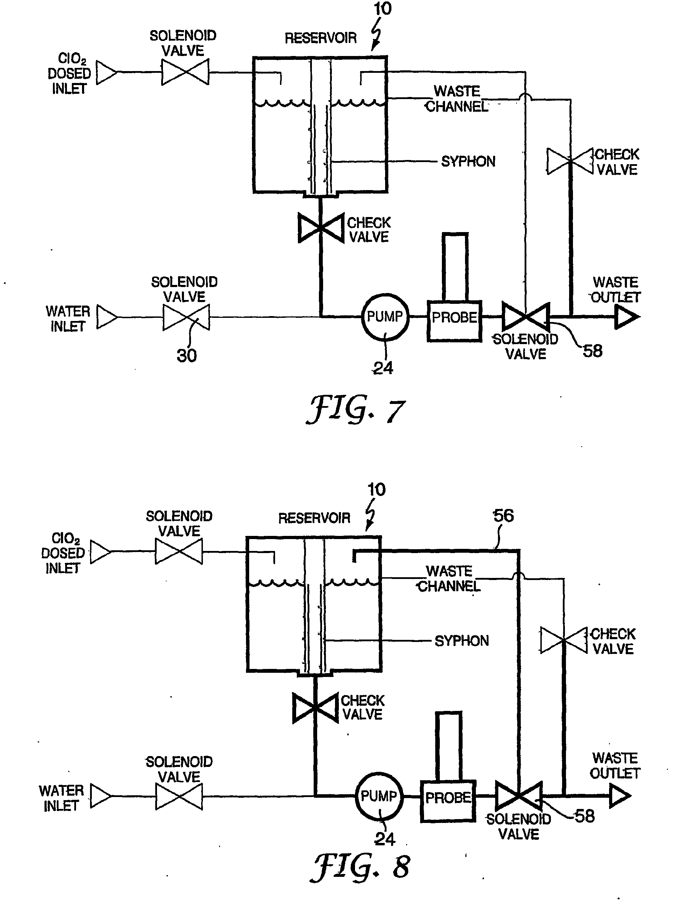

Referring to Figure 7, the second solenoid valve 30 is closed and the third solenoid valve 58 is opened to direct water from the reservoir 10 to waste. The pump 24 is activated to pump the contents of the reservoir 10 to waste for one minute, or for a time period long enough to ensure that the reservoir 10 is completely emptied.

### Stage 7: Empty tubes

Referring to Figure 8, the third solenoid valve 58 is set to direct fluid to the reservoir 10. The pump 24 is activated to pump a slug of water in the return channel 56 into the reservoir 10. The third solenoid valve 58 is then set to direct fluid to waste, and the pump 24 is activated to pump the slug of water out of the reservoir 10 to waste. At this stage, all of the system tubes are empty.

### Stage 8: Ensure probe is left immersed in plain water

Referring to Figure 9, the second solenoid valve 28 is opened and water is flushed through the pump 24 and the probe 22 to waste. The pump 24 is stopped, and the second solenoid valve 28 is closed. The third solenoid valve 58 is set to direct water to the reservoir 10. Since the pump is not activated at this stage, and the second solenoid valve 28 is close, water does not pass into the reservoir 10 through the return channel 56. Instead, the water is trapped in the sample channel 20, which leaves the probe 22 immersed in water. The reservoir 10 is now empty and ready for the next refill and sample cycle to begin.

We have found that, with the chemical compositions used, agitation increases the measured ClO₂ concentration in a sample. When a sample is first mixed the measured ClO₂ concentration falls quite rapidly if the sample is not agitated. The measured concentration rises if the sample is agitated. If we agitate a mixed sample by hand, and each time take a measurement, we find that the measurement varies. If we agitate the sample using the pump, the agitation is much more consistent, as the speed of the motor and the time the motor is operated for are software controlled, and the measured Cl0₂ concentration is much more consistent.

Thus, the consistency of the measured result is influenced by the agitation of the fluid through the pump. The exemplified system uses a consistent agitation and produces very consistent results.

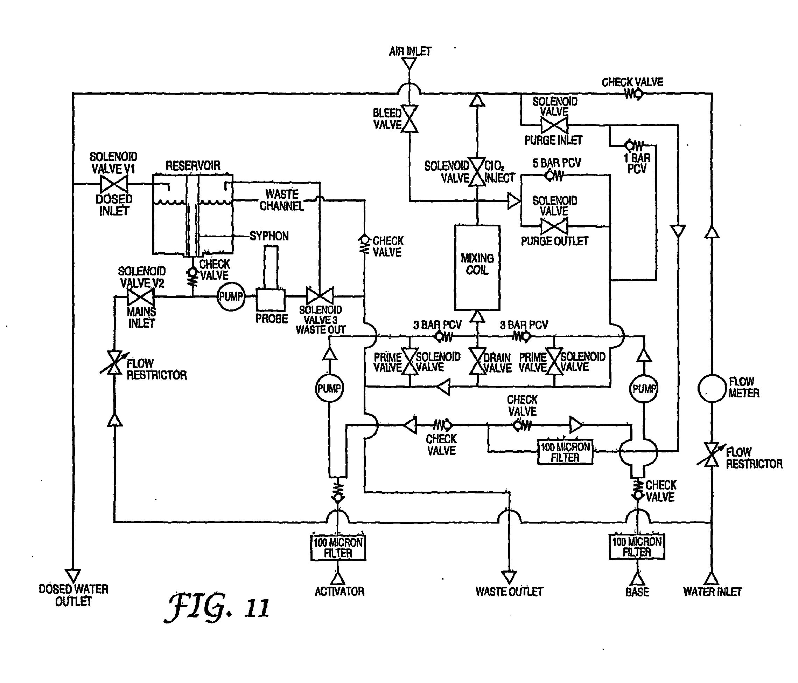

A chlorine dioxide generator incorporating the apparatus of Figure 1 is illustrated schematically in the flow sheet of Figure 11. The generator is suitable for producing a stream of aqueous chlorine dioxide (ClO₂) sterilizing fluid for use in sterilizing medical and dental instruments or for wound irrigation or skin asepsis. The -concentration of ClO₂ will be varied according the intended application. The method of generating ClO₂ is similar to that described in WO2005/011759 A1 with respect to Figure 5 therein.

The apparatus comprises a first pump connected to a source of base and a second pump connected to a source of activator. The therms 'base' and 'activator' are used herein to refer to fluids which when mixed react to produce aqueous ClO₂. The reaction mixture preferably has a pH in the range 4.5 to 6.5, notably 5.5 to 6.5. In this example, the base and activator are comprised as set forth in Example 3 of US 5,696,046.

The output from each pump connects to the proximal end of a tubular helical coil (ClO₂ mixing coil) which functions as an elongate reaction chamber. As pumping continues, reaction mixture is pumped progressively through the mixing coil to the distal end of the coil. With pumping continuing, the reaction mixture passes from through the mixing coil in about 30 seconds, during which time the reaction to form ClO₂ is substantially complete. With further pumping, the reaction mixture is injected into a conduit through which passes a stream of water. The reaction mixture mixes with the water to form a stream of aqueous ClO₂ sterilizing fluid. Depending on the rate of pumping, the concentration of ClO₂ in the sterilizing fluid can, in this example, be varied between about 20 ppm and about 200 ppm. Concentrations above or below this range may be generated either by varying the pumping rate, the water flow rate, or the concentration of reagents.

In this example each pump is a diaphragm pump, although other types of pump, notably piston pumps, could be used. The diaphragm pumps use an elastomeric diaphragm to draw fluid in and to expel fluid from the pumping chamber. On the downstroke the diaphragm draws fluid into the chamber through an inlet valve and on the up-stroke the diaphragm forces fluid out through an outlet valve. The pumps are self-priming. They can provide pumping pressures up to 600 kPa and flow rates in the range 0 to 300 ml/min. Advanced motor control technology speeds up the motor on the intake stroke to minimize fluid output pulsing. In this example, the pump body and valve components are made from stainless steel, and the diaphragms are coated with PTFE. A variety of motor and encoder options are available to drive and control operation of the pumps. In the present example, the pumps are operated under stepper motor control and monitored by means of optical encoders mounted on the motor shafts. The optical encoders measure graticules as they pass the optical sensor, giving precise measurement of shaft rotation, and hence volume pumped. The displacement volume for each pump per cycle is about 0.5 ml.

Each pump is provided with a pressure regulator (pressure control valve), which ensures that pumping is carried out against a substantially constant back pressure. The pressure control valve comprises a spring-biased piston against which the pump works. This arrangement ensures that the pump operates at a constant pressure regardless of the pressure in the water supply. To increase the working life of the pressure control valves it is preferred that the valve fittings are coated with PTFE or a fluoroelastomeric material which has good resistance to chemical attack. In the present example example, the back pressure is 4 bar (400 kPa), which is the same as the nominal mains water pressure in the conduit. All pumps are affected by back-pressure, which reduces the flow rate. By setting a constant back pressure, the pressure experienced by the pumps is kept constant, which in turn keeps the volume expelled during each pump cycle substantially constant. Thus, the apparatus enables precisely controlled quantities of reagents to be mixed and injected into the water stream, thereby providing a stream of sterilizing fluid with the concentration of ClO₂ controlled to within the limits demanded by particular applications within a medical or surgical facility. In a preferred embodiment, the spring pressure may be adjusted if desired, to permit corresponding adjustment to the bolus volume delivered by the pump.

PTFE tube connections are used to connect the pump outputs to the mixing coil, with stainless steel fittings used to provide greater resistance to damage from ClO₂. The valves are stainless steel solenoid valves with Viton^{®} fluoroelastomer fittings. Suitable valves are sold by Parker Hannifin and Cole Palmer.

The mixing coil in this example is a vertically-mounted stainless steel helical tube. The volume capacity of the coil is selected according to the intended application. For most applications, the mixing coil will have a 20 ml capacity. However, for alternative product variants, the coil may have a greater capacity, for example 100 ml capacity, based on a preferred mixing time of 30 seconds. The tubing which carries the activator and base solutions to their respective pumps is provided with a 100 µm filter and a check valve to ensure one-way travel of fluid. The ClO2 solution is injected into the mains water supply via a solenoid valve. If air is initially in the system this may be bled off via a bleed valve.

In this embodiment, the dosed water outlet line has a check valve, which ensures that water will not pass through until a preset pressure of 100 kPa is reached. This arrangement ensures that the system is always pressurized when in use.

The system includes an arrangement for priming and purging the system. Priming the system involves initially filling the diaphragm pumps with water so that they can operate on liquids, which they pump efficiently, and not air, which can cause diaphragm pumps problems. Purging the system involves flushing aqueous ClO₂ out with water to prevent long-term contact of the internal surfaces with ClO₂, which is desirable to increase the operating lifetime of the system. To initiate purging, the solenoid purge valve is opened, permitting water to pass through a purge line to the tubing which connects the base and activator sources to the diaphragm pumps. The purge water passes through a 100 µm filter, and check valves ensure that the purge water passes up through the pumps and into the mixing coil. With the ClO₂ injection solenoid valve closed, and a purge outlet solenoid valve open, purge water passes from the coil and leaves through a waste outlet. To prime the pumps without purging the coil, the solenoid prime valves are opened so that water which passes through the pumps goes directly to the waste outlet. A 100 kPa check valve connects the output from the purge inlet to waste, thereby limiting purge water pressure. Similarly, a 500 kPa check valve is provided to vent redaction products from the mixing coil to waste to prevent dangerous excess of pressure if the ClO₂ injection valve and the purge outlet valves are closed. A drain valve is provided to permit manual draining of the coil to waste.

### Example 1:

The washer needs 40 ml of ClO₂ solution from the mixing coil to dose the rinse water of the 15 litre sink at the rate of 40 ml/minute. Base and Activator are injected into the base of the mixing coil by the two pumps. The internal volume of the coil is 20 ml. It takes 30 seconds for the Base and Activator to fill the coil. During the 30 seconds the Base and Activator are thoroughly mixed and ClO₂ is produced. The Base and Activator pump -continue to push the 20 ml packet of ClO₂ out of the mixing coil and into the water line at 400 kPa at the demand rate of 40 ml/minute. At the end of the delivery cycle the mixing coil is still full of ClO₂. Software control may be used to determine whether to hold this ClO₂ in the coil, because there is an expectation that another batch will be required in a pre-determined period, or whether to flush it out using Base or water.

### Example 2:

The washer needs 160 ml of ClO₂ solution to dose both chambers of a washer disinfection machine at the rate of 40 ml/minute. We proceed exactly as Example 1 above but continue to mix and pump ClO₂ at the rate of 40 ml/minute until 160 ml of ClO₂ has been dispensed into the water supply. At the end of the delivery cycle the mixing coil can be flushed with Base or water or the ClO₂ retained as required.

### Verification of ClO₂ concentration using probe technology

The flow control apparatus of Figure 1 inputs ClO₂ dosed water from the water supply line downstream of the point where the reaction mixture from the mixing coil is injected into the water stream. The fresh water feed to the probe system in this example is taken from the upstream side of the water inlet flow meter. To ensure that the fresh water feed to the probe can be closed for servicing, solenoid valve V2 is provided at the base of the reservoir. This arrangement has the benefit that when fresh water is drawn into the probe manifold system to flush the reservoir, the flow meter does not detect it and the Generator is not activated.

In an alternative preferred configuration the fresh water feed to the probe would be located between the flow restrictor outlet and the flow meter inlet so that when the regulator is closed the water supply to both the Generator and the probe may be isolated from the mains.

As described above with reference to Figures 1-10, the syphon system permits the probe to measure the concentration of ClO₂ in the dosed water, and then to flush the probe with fresh water which is subsequently pumped to a waste outlet.

This aspect of the invention provides a chlorine dioxide generator with means for accurately measuring ClO₂ concentration when desired.

Timings for flushing of the probe may be implemented in software. The timings may be varied for different applications and component sizes; examples of timings are given below.

Duration of ProbeFlush() FLUSH1 state, in which the sample tank is emptied: FLUSH1_TIME=75 secs

Duration of ProbeFlush() FLUSH2 state, in which pump and probe is flushed with clean water: FLUSH2_TIVE=5 secs

Duration of ProbeFlush() FLUSH3 state, in which the sample tank is filled with clean water: FLUSH3_TIME=30 secs

Duration of ProbeFlush() FLUSH4 state, in which the sample tank is emptied of clean water: FLUSH4_TIME=75 secs

Duration of ProbeFlush() FLUSH4A state, in which the slug of fluid in the recirculation tubing is transferred to the sample tank: FLUSH4A_TIME=5 secs

Duration of ProbeFlush() FLUSH4B state, in which the slug of fluid in the sample tank is emptied to waste: FLUSH4B_TIME=5 secs

Duration of ProbeFlush() FLUSH5 state, in which pump and probe is flushed with clean water: FLUSH5_TIME=2 secs

Duration of recirculation time, in which dosed water is passed across the probe. This duration is determined by the response time of the probe: RECIRCULATE_TIME=120 secs

It should be understood that the invention has been described by way of example only and that modifications in detail may be made without departing from the scope of the invention as specified in the claims.

## Claims

1. Apparatus for controlling the flow of fluids through a sample channel (20), the apparatus comprising:
a reservoir (10) having a reservoir inlet (12), the reservoir inlet (12) being for connection to a source of a first fluid;
a sample channel (20) having a sample channel inlet (28), the sample channel inlet (28) being for connection to a source of a second fluid; **characterised by**:
a syphon (32) connected between the reservoir (10) and the sample channel (20);
means (30,58) for trapping fluid in at least part of the sample channel (20);
wherein the apparatus is configured such that in use air can be allowed into the syphon (32) to create an air gap in the syphon, the air gap preventing first fluid in the reservoir (10) from contacting trapped fluid in the sample channel (20);
the apparatus further comprising pump means (24) for pumping fluid out of the sample channel (20) and for pumping air out of the syphon (32) such that fluid in the reservoir (10) can flow through the syphon (32) and into the sample channel (20).

2. Apparatus as claimed in Claim 1, wherein the syphon (32) comprises an inner portion (36) and an outer portion (38), the outer portion having a closed end (46) located near the top of the reservoir, and an open end (48) located near the bottom of the reservoir, the inner portion (36) being located within the outer portion (38) and surrounding the reservoir outlet (14), the inner portion having sidewalls (42) extending from the bottom of the reservoir towards the top of the reservoir.

3. Apparatus as claimed in Claim 2, wherein the inner (36) and outer (38) portions are concentric cylinders.

4. Apparatus as claimed in Claim 2 or Claim 3, wherein the reservoir (10) further comprises fluid level control means (62) for controlling the level of fluid in the reservoir such that the fluid level does not exceed a maximum fill level.

5. Apparatus as claimed in Claim 4, wherein the fluid level control (62) means comprises an overflow outlet.

6. Apparatus as claimed in Claim 4 or Claim 5, wherein the height of the sidewalls (42) of the syphon inner portion (36) are substantially equal to or greater than the maximum fill level.

7. Apparatus as claimed in any preceding claim, further comprising a probe (22) located within the sample channel (20) for analysing a property of a fluid from the reservoir.

8. Apparatus as claimed in Claim 7, wherein the probe (22) is a probe for measuring the concentration of chlorine dioxide in an aqueous fluid.

9. Apparatus as claimed in Claim 7 or Claim 8, further comprising a return channel (56) connected between the sample channel (20) and the reservoir (10), the return channel (56) being connected to the sample channel (20) downstream of the probe (22).

10. Apparatus as claimed in Claim 9, wherein a solenoid valve (58) is located between the sample channel (20) and the return channel (56) to selectively direct fluid from the sample channel (20) through the return channel (56) or towards a waste outlet (60).

11. Apparatus as claimed in any preceding claim, wherein the pump means includes a pump (24) located in the sample channel (20).

12. Apparatus as claimed in any preceding claim, wherein the pump means is a single pump (24).

13. Apparatus as claimed in Claim 12, wherein the single pump (20) may be used for pumping the first and second fluids through the sample channel (20), through the return channel (56), and through the reservoir (10).

14. Apparatus as claimed in any preceding claim, wherein a check valve (54) is located between the sample channel (20) and the reservoir (10) to prevent fluid from passing from the sample channel into the reservoir.

15. Apparatus as claimed in any preceding claim, wherein a solenoid valve (17) is located between the source of the first fluid (18) and the reservoir (10) for selectively allowing fluid to enter the reservoir.

16. Apparatus as claimed in any preceding claim, further including a solenoid valve (30) for selectively allowing the second fluid to flow through the sample channel (20).

17. Apparatus as claimed in any preceding claim, wherein the means for trapping fluid in at least part of the sample channel comprises two solenoid valves (30,58) located at either end of said part of the sample channel (20).

18. Apparatus as claimed in any preceding claim, wherein the reservoir (10) is a sealed unit.

19. Apparatus as claimed in Claim 18, wherein the reservoir (10) has a duckbilled valve.

20. A method of controlling the flow of fluids through a sample channel (20), the method comprising the steps of:
providing a reservoir (10);
providing a sample channel (20); **characterised by**:
providing a syphon (32) connecting the reservoir (10) and the sample channel (20);
trapping a second fluid in at least a part of the sample channel (20);
allowing the syphon (32) to fill with air;
filling the reservoir (10) with a first fluid;
the air in the syphon preventing the first fluid in the reservoir from entering the sample channel (20) and contacting the trapped second fluid;
providing pump means (24);
pumping second fluid out of the sample channel (20);
pumping air out of the syphon (32) and pumping at least some of the first fluid out of the reservoir (10) and through the syphon (32) into the sample channel (20).

21. A method according to claim 20, further comprising measuring the concentration of a component of a fluid in the sample channel (20) by:
providing a probe (22) located in the sample channel (20);
submersing at least part of the probe (22) in the second fluid when the second fluid is trapped in the sample channel;
and
using the probe to measure the concentration of a component of the first fluid when the first fluid has been pumped through the syphon (32) into the sample channel (20).

22. A method as claimed in Claim 21, further comprising the steps of:
providing a return channel (56) connected between the sample channel (20) and the reservoir (10), the return channel (56) being connected to the sample channel (20) downstream of the probe (22);
directing the first fluid along the return channel (56) and back into the reservoir (10);
pumping the recirculated first fluid from the reservoir (10) and through the sample channel (20) and past the concentration probe (22);
continuing the above steps for a predetermined time period;
pumping the contents of the reservoir (10) to waste after the predetermined time period.

23. A method as claimed in Claim 22, wherein the predetermined sample time period is approximately 2 minutes.

24. A method as claimed in Claim 22 or Claim 23, further comprising the steps of:
providing a valve (30) to selectively allow second fluid to flow through the sample channel (20); and
providing a valve (58) to selectively direct fluid from the sample channel (20) to the return channel (56);
wherein the step of trapping second fluid in the sample channel (20) is achieved by setting the valves so that water is prevented from entering the sample channel (20), and so that fluid in the sample channel (20) is directed to the return channel (56).

25. A method as claimed in any one of Claims 21 to 24, further comprising the step of providing verifying means to automatically check whether the measured concentration value is within a predetermined tolerance of a desired concentration value.

26. A method as claimed in Claim 25, further comprising the step of providing display means for displaying a pass or fail notice to indicate whether the concentration is within the predetermined tolerance or not.

## Patentansprüche

1. Vorrichtung zum Steuern der Strömung von Fluiden durch einen Probenkanal (20), wobei die Vorrichtung Folgendes enthält:
einen Vorratsbehälter (10) mit einem Vorratsbehältereinlass (12), wobei der Vorratsbehältereinlass (12) für die Verbindung mit einer Quelle eines ersten Fluids vorgesehen ist;
einen Probenkanal (20) mit einem Probenkanaleinlass (28), wobei der Probenkanaleinlass (28) für die Verbindung mit einer Quelle eines zweiten Fluids vorgesehen ist; **gekennzeichnet durch**:
ein Saugrohr (32), das zwischen dem Vorratsbehälter (10) und dem Probenkanal (20) verbunden ist;
Mittel (30, 58), um Fluid wenigstens in einem Teil des Probenkanals (20) einzufangen;
wobei die Vorrichtung in der Weise konfiguriert ist, dass im Gebrauch Luft in das Saugrohr (32) gelassen werden kann, um in dem Saugrohr einen Luftspalt zu erzeugen, wobei der Luftspalt verhindert, dass erstes Fluid in dem Vorratsbehälter (10) mit eingefangenem Fluid in dem Probenkanal (20) in Kontakt gelangt;
wobei die Vorrichtung ferner Pumpmittel (24) enthält, um Fluid aus dem Probenkanal (20) zu pumpen und um Luft aus dem Saugrohr (32) zu pumpen, derart, dass Fluid in dem Vorratsbehälter (10) **durch** das Saugrohr (32) und in den Probenkanal (20) strömen kann.

2. Vorrichtung nach Anspruch 1, wobei das Saugrohr (32) einen inneren Abschnitt (36) und einen äußeren Abschnitt (38) aufweist, wobei der äußere Abschnitt ein geschlossenes Ende (46), das sich in der Nähe der Oberseite des Vorratsbehälters befindet, und ein offenes Ende (48), das sich in der Nähe der Unterseite des Vorratsbehälters befindet, enthält, wobei sich der innere Abschnitt (36) in dem äußeren Abschnitt (38) befindet und den Vorratsbehälterauslass (14) umgibt, wobei der innere Abschnitt Seitenwände (42) besitzt, die sich von der Unterseite des Vorratsbehälters zu der Oberseite des Vorratsbehälters erstrecken.

3. Vorrichtung nach Anspruch 2, wobei der innere Abschnitt (36) und der äußere Abschnitt (38) konzentrische Zylinder sind.

4. Vorrichtung nach Anspruch 2 oder Anspruch 3, wobei der Vorratsbehälter (10) ferner Fluidpegel-Steuermittel (62) enthält, um den Fluidpegel in dem Vorratsbehälter zu steuern, so dass der Fluidpegel einen maximalen Befüllungspegel nicht übersteigt.

5. Vorrichtung nach Anspruch 4, wobei die Fluidpegel-Steuermittel (62) einen Überlaufauslass enthalten.

6. Vorrichtung nach Anspruch 4 oder Anspruch 5, wobei die Höhe der Seitenwände (42) des inneren Abschnitts (36) des Saugrohrs im Wesentlichen gleich oder größer als der maximale Befüllungspegel ist.

7. Vorrichtung nach einem vorgehenden Anspruch, die ferner einen Fühler (22) enthält, der sich in dem Probenkanal (20) befindet, um eine Eigenschaft eines Fluids aus dem Vorratsbehälter zu analysieren.

8. Vorrichtung nach Anspruch 7, wobei der Fühler (22) ein Fühler zum Messen der Konzentration von Chlordioxid in einem wässrigen Fluid ist.

9. Vorrichtung nach Anspruch 7 oder Anspruch 8, die ferner einen Rückleitungskanal (56) enthält, der zwischen dem Probenkanal (20) und dem Vorratsbehälter (10) verbunden ist, wobei der Rückleitungskanal (56) mit dem Probenkanal (20) stromabseitig von dem Fühler (22) verbunden ist.

10. Vorrichtung nach Anspruch 9, wobei sich ein Magnetventil (58) zwischen dem Probenkanal (20) und dem Rückleitungskanal (56) befindet, um Fluid wahlweise von dem Probenkanal (20) durch den Rückleitungskanal (56) oder zu einem Abfallauslass (60) zu lenken.

11. Vorrichtung nach einem vorhergehenden Anspruch, wobei die Pumpmittel eine Pumpe (24) enthalten, die sich in dem Probenkanal (20) befindet.

12. Vorrichtung nach einem vorhergehenden Anspruch, wobei die Pumpmittel eine einzige Pumpe (24) sind.

13. Vorrichtung nach Anspruch 12, wobei die einzige Pumpe (20) verwendet werden kann, um erste und zweite Fluide durch den Probenkanal (20), durch den Rückleitungskanal (56) und durch den Vorratsbehälter (10) zu pumpen.

14. Vorrichtung nach einem vorhergehenden Anspruch, wobei sich zwischen dem Probenkanal (20) und dem Vorratsbehälter (10) ein Rückschlagventil (54) befindet, um zu verhindern, dass sich Fluid von dem Probenkanal in den Vorratsbehälter bewegt.

15. Vorrichtung nach einem vorhergehenden Anspruch, wobei ein Magnetventil (17) zwischen der Quelle des ersten Fluids (18) und dem Vorratsbehälter (10) angeordnet ist, um dem Fluid gezielt den Eintritt in den Vorratsbehälter zu gestatten.

16. Vorrichtung nach einem vorhergehenden Anspruch, die ferner ein Magnetventil (30) enthält, um dem zweiten Fluid wahlweise zu erlauben, durch den Probenkanal (20) zu strömen.

17. Vorrichtung nach einem vorhergehenden Anspruch, wobei die Mittel zum Einfangen von Fluid wenigstens in einem Teil des Probenkanals zwei Magnetventile (30, 58) enthalten, die sich an jedem Ende des Teils des Probenkanals (20) befinden.

18. Vorrichtung nach einem vorhergehenden Anspruch, wobei der Vorratsbehälter (10) eine abgedichtete Einheit ist.

19. Vorrichtung nach Anspruch 18, wobei der Vorratsbehälter (10) ein Lippenventil besitzt.

20. Verfahren zum Steuern der Strömung von Fluiden durch einen Probenkanal (20), wobei das Verfahren die folgenden Schritte enthält:
Vorsehen eines Vorratsbehälters (10);
Vorsehen eines Probenkanals (20); **gekennzeichnet durch**:
Vorsehen eines Saugrohrs (32), das den Vorratsbehälter (10) und den Probenkanal (20) verbindet;
Einfangen eines zweiten Fluids wenigstens in einem Teil des Probenkanals (20);
Zulassen, dass das Saugrohr (32) mit Luft gefüllt wird;
Befüllen des Vorratsbehälters (10) mit einem ersten Fluid;
wobei die Luft im Saugrohr verhindert, dass das erste Fluid im Vorratsbehälter in den Probenkanal (20) eintritt und mit dem eingefangenen zweiten Fluid in Kontakt gelangt;
Vorsehen von Pumpmittel (24);
Pumpen von zweitem Fluid aus dem Probenkanal (20);
Pumpen von Luft aus dem Saugrohr (32) und Pumpen wenigstens eines Teils des ersten Fluids aus dem Vorratsbehälter (10) und **durch** das Saugrohr (32) in den Probenkanal (20).

21. Verfahren nach Anspruch (20), das ferner das Messen der Konzentration einer Komponente eines Fluids in dem Probenkanal (20) enthält, indem:
ein Fühler (22) vorgesehen wird, der sich in dem Probenkanal (20) befindet;
wenigstens ein Teil des Fühlers (22) in dem zweiten Fluid untergetaucht wird, wenn das zweite Fluid in dem Probenkanal eingefangen ist; und
der Fühler verwendet wird, um die Konzentration einer Komponente des ersten Fluids zu messen, wenn das erste Fluid durch das Saugrohr (32) in den Probenkanal (20) gepumpt worden ist.

22. Verfahren nach Anspruch 21, das ferner die folgenden Schritte enthält:
Vorsehen eines Rückleitungskanals (56), der zwischen dem Probenkanal (20) und dem Vorratsbehälter (10) verbunden ist, wobei der Rückleitungskanal (56) mit dem Probenkanal (20) stromabseitig von dem Fühler (22) verbunden ist;
Lenken des ersten Fluids längs des Rückleitungskanals (56) und zurück in den Vorratsbehälter (10);
Pumpen des zurückgeleitenden ersten Fluids aus dem Vorratsbehälter (10) und durch den Probenkanal (20) und an dem Konzentrationsfühler (22) vorbei;
Fortsetzen der obigen Schritte für eine vorgegebene Zeitdauer;
Pumpen der Inhalte des Vorratsbehälters (10) zu Abfall nach der vorgegebenen Zeitdauer.

23. Verfahren nach Anspruch 22, wobei die vorgegebene Probenzeitdauer etwa 2 Minuten beträgt.

24. Verfahren nach Anspruch 22 oder Anspruch 23, das ferner die folgenden Schritte enthält:
Vorsehen eines Ventils (30), um wahlweise zuzulassen, dass zweites Fluid durch den Probenkanal (20) strömt; und
Vorsehen eines Ventils (58), um wahlweise Fluid von dem Probenkanal (20) zu dem Rückleitungskanal (56) zu lenken;
wobei der Schritt des Einfangens von zweitem Fluid im Probenkanal (20) durch Einstellen der Ventile in der Weise erzielt wird, dass verhindert wird, dass Wasser in den Probenkanal (20) eindringt, und dass Fluid im Probenkanal (20) zu dem Rückleitungskanal (56) gelenkt wird.

25. Verfahren nach einem der Ansprüche 21 bis 24, das ferner den Schritt des Vorsehens von Verifikationsmitteln enthält, um automatisch zu prüfen, ob der gemessene Konzentrationswert innerhalb einer vorgegebenen Toleranz eines Soll-Konzentrationswerts liegt.

26. Verfahren nach Anspruch 25, das ferner den Schritt des Vorsehens von Anzeigemitteln zum Anzeigen einer Bestanden- oder Nichtbestanden-Meldung enthält, um anzugeben, ob die Konzentration innerhalb der vorgegebenen Toleranz liegt oder nicht.

## Revendications

1. Appareil pour réguler le flux des fluides dans un canal d'échantillon (20), l'appareil comprenant :
un réservoir (10) ayant une entrée de réservoir (12), l'entrée de réservoir (12) étant prévue pour le raccordement à une source d'un premier fluide ;
un canal d'échantillon (20) ayant une entrée de canal d'échantillon (28), l'entrée de canal d'échantillon (28) étant prévue pour le raccordement à une source d'un second fluide ; **caractérisé par** :
un siphon (32) raccordé entre le réservoir (10) et le canal d'échantillon (20) ;
des moyens (30, 58) pour piéger le fluide dans au moins une partie du canal d'échantillon (20) ;
dans lequel l'appareil est configuré de sorte qu'à l'usage, l'air peut être autorisé dans le siphon (32) afin de créer un espace d'air dans le siphon, l'espace d'air empêchant le premier fluide dans le réservoir (10) d'entrer en contact avec le fluide piégé dans le canal d'échantillon (20) ;
l'appareil comprenant en outre des moyens de pompe (24) pour pomper le fluide hors du canal d'échantillon (20) et pour pomper l'air hors du siphon (32) de sorte que le fluide dans le réservoir (10) peut s'écouler à travers le siphon (32) et dans le canal d'échantillon (20).

2. Appareil selon la revendication 1, dans lequel le siphon (32) comprend une partie interne (36) et une partie externe (38), la partie externe ayant une extrémité fermée (46) située à proximité de la partie supérieure du réservoir, et une extrémité ouverte (48) situé à proximité du fond du réservoir, la partie interne (36) étant positionnée à l'intérieur de la partie externe (38) et entourant la sortie de réservoir (14), la partie interne ayant des parois latérales (42) s'étendant du fond du réservoir vers la partie supérieure du réservoir.

3. Appareil selon la revendication 2, dans lequel les parties interne (36) et externe (38) sont des cylindres concentriques.

4. Appareil selon la revendication 2 ou la revendication 3, dans lequel le réservoir (10) comprend en outre des moyens de régulation de niveau de fluide (62) pour réguler le niveau de fluide dans le réservoir de sorte que le niveau de fluide ne dépasse pas un niveau de remplissage maximum.

5. Appareil selon la revendication 4, dans lequel les moyens de régulation de niveau de fluide (62) comprennent une sortie de trop-plein.

6. Appareil selon la revendication 4 ou la revendication 5, dans lequel la hauteur des parois latérales (42) de la partie interne (36) du siphon est sensiblement égale ou supérieure au niveau de remplissage maximum.

7. Appareil selon l'une quelconque des revendications précédentes, comprenant en outre une sonde (22) positionnée à l'intérieur du canal d'échantillon (20) pour analyser une propriété d'un fluide du réservoir.

8. Appareil selon la revendication 7, dans lequel la sonde (22) est une sonde pour mesurer la concentration de dioxyde de chlore dans un fluide aqueux.

9. Appareil selon la revendication 7 ou la revendication 8, comprenant en outre un canal de retour (56) interconnecté entre le canal d'échantillon (20) et le réservoir (10), le canal de retour (56) étant raccordé au canal d'échantillon (20) en aval de la sonde (22).

10. Appareil selon la revendication 9, dans lequel une électrovanne (58) est positionnée entre le canal d'échantillon (20) et le canal de retour (56) pour diriger sélectivement le fluide du canal d'échantillon (20) en passant par le canal de retour (56) ou bien vers une sortie de déchets (60).

11. Appareil selon l'une quelconque des revendications précédentes, dans lequel les moyens de pompe comprennent une pompe (24) positionnée dans le canal d'échantillon (20).

12. Appareil selon l'une quelconque des revendications précédentes, dans lequel les moyens de pompe sont une seule pompe (4).

13. Appareil selon la revendication 12, dans lequel la pompe unique (20) peut être utilisée pour pomper les premier et second fluides à travers le canal d'échantillon (20), à travers le canal de retour (56) et à travers le réservoir (10).

14. Appareil selon l'une quelconque des revendications précédentes, dans lequel un clapet antiretour (54) est positionné entre le canal d'échantillon (20) et le réservoir (10) pour empêcher le fluide de passer du canal d'échantillon dans le réservoir.

15. Appareil selon l'une quelconque des revendications précédentes, dans lequel une électrovanne (17) est positionnée entre la source du premier fluide (18) et le réservoir (10) pour permettre sélectivement au fluide d'entrer dans le réservoir.

16. Appareil selon l'une quelconque des revendications précédentes, comprenant en outre une électrovanne (30) pour permettre sélectivement au second fluide de s'écouler à travers le canal d'échantillon (20).

17. Appareil selon l'une quelconque des revendications précédentes, dans lequel les moyens pour piéger le fluide dans au moins une partie du canal d'échantillon comprend deux électrovannes (30, 58) positionnées à chaque extrémité de ladite partie du canal d'échantillon (20).

18. Appareil selon l'une quelconque des revendications précédentes, dans lequel le réservoir (10) est une unité étanche.

19. Appareil selon la revendication 18, dans lequel le réservoir (10) a un clapet en bec de canard.

20. Procédé pour réguler le flux des fluides à travers un canal d'échantillon (20), le procédé comprenant les étapes consistant à :
prévoir un réservoir (10) ;
prévoir un canal d'échantillon (20) ; **caractérisé par** les étapes consistant à :
prévoir un siphon (32) raccordant le réservoir (10) et le canal d'échantillon (20) ;
piéger un second fluide dans au moins une partie du canal d'échantillon (20) ;
permettre au siphon (32) de se remplir avec de l'air ;
remplir le réservoir (10) avec un premier fluide ;
l'air dans le siphon empêchant le premier fluide dans le réservoir d'entrer dans le canal d'échantillon (20) et d'être en contact avec le second fluide piégé ;
prévoir des moyens de pompe (24) ;
pomper le second fluide hors du canal d'échantillon (20) ;
pomper l'air hors du siphon (32) et pomper au moins une certaine partie du premier fluide hors du réservoir (10) et à travers le siphon (32) dans le canal d'échantillon (20).

21. Procédé selon la revendication 20, comprenant en outre l'étape consistant à mesurer la concentration d'un composant d'un fluide dans le canal d'échantillon (20) avec les étapes consistant à :
prévoir une sonde (22) positionnée dans le canal d'échantillon (20) ;
immerger au moins une partie de la sonde (22) dans le second fluide lorsque le second fluide est piégé dans le canal d'échantillon ;
et
utiliser la sonde pour mesurer la concentration d'un composant du premier fluide lorsque le premier fluide a été pompé par le siphon (32) dans le canal d'échantillon (20).

22. Procédé selon la revendication 21, comprenant en outre les étapes consistant à :
prévoir un canal de retour (56) raccordé entre le canal d'échantillon 20) et le réservoir (10), le canal de retour (56) étant raccordé au canal d'échantillon (20) en aval de la sonde (22) ;
diriger le premier fluide le long du canal de retour (56) et le faire revenir dans le réservoir (10) ;
pomper le premier fluide recirculé du réservoir (10) et en passant par le canal d'échantillon (20 et au-delà de la sonde de concentration (22) ;
continuer les étapes ci-dessus pendant une période de temps prédéterminée ;
pomper le contenu du réservoir (10) pour le jeter après la période de temps prédéterminée.

23. Procédé selon la revendication 22, dans lequel la période de temps d'échantillon prédéterminée est d'approximativement 2 minutes.

24. Procédé selon la revendication 22 ou la revendication 23, comprenant en outre les étapes consistant à :
prévoir une soupape (30) pour permettre sélectivement au second fluide de s'écouler à travers le canal d'échantillon (20) ; et
prévoir un clapet (58) pour diriger sélectivement le fluide du canal d'échantillon (20) au canal de retour (56) ;
dans lequel l'étape consistant à piéger le second fluide dans le canal d'échantillon (20) est obtenue en réglant les clapets de sorte que l'on empêche l'eau d'entrer dans le canal d'échantillon (20) et de sorte que le fluide dans le canal d'échantillon (20) est dirigé vers le canal de retour (56).

25. Procédé selon l'une quelconque des revendications 21 à 24, comprenant en outre l'étape consistant à prévoir des moyens de vérification pour vérifier automatiquement si la valeur de concentration mesurée est dans une tolérance prédéterminée d'une valeur de concentration souhaitée.

26. Procédé selon la revendication 25, comprenant en outre l'étape consistant à prévoir des moyens d'affichage pour afficher une instruction d'autorisation ou d'erreur pour indiquer si la concentration est dans la tolérance prédéterminée ou pas.
